# EUROPEAN PATENT SPECIFICATION

(11) **EP 3 111 971 B1**
(45) Date of publication and mention of the grant of the patent: **09.12.2020**
(21) Application number: 15754993.2
(22) Date of filing: 18.02.2015
(51) Int. Cl.: A61M 1/02

(54) **SUSPENSION JIG AND METHOD FOR TRANSPORTING ADDITIVE SOLUTION**
HÄNGETRÄGER UND VERFAHREN ZUM TRANSPORT EINER ADDITIVLÖSUNG
POTENCE À SUSPENSION ET PROCÉDÉ DE TRANSPORT DE SOLUTION D'ADDITIF

(30) Priority: 26.02.2014 JP 2014035141
(43) Date of publication of application: 04.01.2017
(73) Proprietor: Terumo Kabushiki Kaisha, Tokyo 151-0072 (JP)
(72) Inventor: IMAI, Tadashi, 3001 Leuven (BE)
(74) Representative: Casalonga
(86) International application number: PCT/JP2015/054418
(87) International publication number: WO 2015/129520

(56) References cited:
- WO-A1-2004/101034
- JP-A- 2002 541 930
- JP-A- 2005 124 813
- US-A- 3 177 870
- US-A- 3 664 814
- US-A- 4 258 723
- US-A- 4 267 837
- US-A- 4 977 850
- US-A- 5 000 407

## Description

### Technical Field

The present invention relates to a hanging device and an additive solution transfer method for a blood bag system.

### Background Art

Transfusion conducted in recent years includes component transfusion, in which a component is separated from blood (whole blood) obtained by blood donation, or the like, and solely a We component required by a patient is supplied. With component transfusion, it is possible to reduce the burden and side effects on a circulatory system of the patient and to effectively use the donated blood.

The blood (whole blood) obtained by blood donation or a blood component prepared from the whole blood is separated through centrifugation into a plurality of layers inside a blood bag. For example, when residual components (white-cell-poor platelet-poor blood) obtained by removing while blood cells and platelets from whole blood are separated into plasma and red blood cells (concentrated red blood cells) and collected into separate bags, the components are initially separated into a plasma layer as a supernatant component and red blood cells (concentrated red blood cells) layer as a sedimentary component by centrifugation (centrifugation step). Thereafter, the plasma is transferred to a plasma bag via a tube connected to the blood bag, so as to leave concentrated red blood cells in the blood bag (separation-transfer step). Next, a red blood cell preservation liquid is added to the concentrated red blood cells by transferring, viaatube, the red blood cell preservation liquid stored in a medical fluid bag into the blood bag (addition step) .

Processing of centrifuging the white-cell-poor platelet-poor blood and collecting the plasma and concentrated red blood cells obtained by centrifugation into separate bags (above-described centrifugation step and separation-transfer step) can be performed automatically by a centrifugation and separation apparatus disclosed in, for example, US patent 6910998 B, and JP 2012-510298 A. In a case where the centrifugation and separation apparatus is used, the above-described addition step is performed manually after the blood bag system is taken out of the centrifugation and separation apparatus. Specifically, by arranging the medical fluid bag at an upper position and the blood bag at a lower position, the red blood cell preservation liquid is transferred to the blood bag using head.

In the above-described addition step, however, when the red blood cell preservation liquid is transferred to the blood bag, not only the red blood cell preservation liquid but also the air present in the medical fluid bag flows into the blood bag. Since predetermined amount of air or more existing in the blood bag might adversely affect red blood cells, there is a quality standard, in some cases, to specify the amount of air to be at a level below the predetermined value . To achieve this, it is necessary to perform air removing operation manually after the addition step, leading to an increased work load.

### Summary of Invention

The present invention is made in view of this issue and an object of the present invention is to provide a hanging device and method for transferring an additive solution capable of efficiently produce blood products at a production process of the blood products by eliminating the need for manual air removing operation after addition of additive solution.

In order to achieve the above-described object, the present invention provides a hanging device configured to hang a blood bag system including: a first bag containing a blood component; a second bag containing additive solution; and a transfer line that forms a flow path between the first bag and the second bag, the hanging device including: a second bag hanging section configured to hang the second bag such that a influx portion of the second bag faces downward; and support mechanisms configured to support the first bag or the transfer line in a predetermined state such that, when the additive solution is transferred from the second bag to the first bag using a height difference between the first bag and the second bag, air from the second bag stops in the middle of the flow path.

With the hanging device configured as above, when the additive solution is transferred from the second bag to the first bag using the height difference between the first bag and the second bag so as to add the additive solution to the blood component in the first bag, the air from the second bag stops in the middle of the transfer line. With this configuration, it is possible to prevent the air from the second bag from mixing into the first bag. Accordingly, after the additive solution is added to the blood component in the first bag, the amount of air in the first bag is the amount that scarcely needs air bleeding operation. As a result, it is possible to eliminate air bleeding operation after addition of the additive solution.

The above-described hanging device may also be configured such that the support mechanism hangs the first bag to arrange the influx portion of the first bag to face downward at a position lower than the second bag hung by the second bag hanging section. By hanging the first bag upside down, the liquid level inside the first bag comes at a position higher than the influx portion of the first bag when the additive solution is transferred from the second bag to the first bag. Accordingly, the air moving inside the transfer line from the second bag stops at a position of the liquid level inside the first bag. As a result, it is possible, with a simple configuration, to appropriately prevent the air from the second bag from mixing into the first bag.

The above-described hanging device may also be configured such that the support mechanism supports the first bag diagonally with respect to the horizontal direction, to arrange the first bag to be gradually lowered toward the influx portion of the first bag at a position lower than the second bag hung by the second bag hanging section. By arranging the first bag diagonally with the influx portion facing downward, the liquid level inside the first bag comes at a position higher than the influx portion of the first bag when the additive solution is transferred from the second bag to the first bag. Accordingly, the air moving inside the transfer line from the second bag stops at a position of the liquid level inside the first bag. As a result, it is possible, with a simple configuration, to appropriately prevent the air from the second bag from mixing into the first bag.

The above-described hanging device may be configured such that the transfer line includes a first bag-side tube formed of a flexible material connected to the first bag, a second bag-side tube formed of a flexible material connected to the second bag, and a branch section formed to be harder than the first bag-side tube and the second bag-side tube and provided between the first bag-side tube and the second bag-side tube, and such that the support mechanism arranges the liquid level inside the first bag to come at a position lower than the branch section. With this configuration, when the transfer line is squeezed to move the additive solution remaining in the flow path of the transfer line to the first bag, it is sufficient to squeeze the first bag-side tube alone. Since the branch section does not hinder the operation, it is possible to smoothly perform the squeezing operation. Furthermore, it is sufficient that the first bag-side tube alone is sealed and cut, and thus, the work would be simple.

The above-described hanging device may be also configured such that the support mechanism supports the first bag and the transfer line so as to form, in the transfer line, a high-level site existing at a position higher than the liquid level inside the first bag, and a low-level site existing at a position lower than the high-level site on both sides of the flow path of the high-level site. With this configuration, when the additive solution is transferred from the second bag to the first bag, the air moving inside the transfer line from the second bag stops at a height of the high-level site. As a result, it is possible, with a simple configuration, to appropriately prevent the air from the second bag from mixing into the first bag.

The above-described hanging device may be configured such that the transfer line includes a first bag-side tube formed of a flexible material, connected to the first bag, a second bag-side tube formed of a flexible material connected to the second bag, and a branch section formed to be harder than the first bag-side tube and the second bag-side tube and provided between the first bag-side tube and the second bag-side tube, and such that the support mechanism arranges the high-level site to come at a position lower than the branch section.

With this configuration, when the transfer line is squeezed to move the additive solution remaining in the tube forming the transfer line to the first bag, it is sufficient to squeeze the first bag-side tube alone. Since the branch section does not hinder the operation, it is possible to smoothly perform the squeezing operation. Furthermore, it is sufficient that the first bag-side tube alone is sealed and cut, and thus, the work would be simple.

The present invention according to the method for transferring the additive solution is targeted at a blood bag system including a first bag containing a blood component, a second bag containing additive solution, and a delivery line that forms a flow path between the first bag and the second bag, the method being configured to transfer the additive solution from the second bag to the first bag, the method for transferring the additive solution including: a support step of supporting the first bag or the transfer line in a predetermined state such that, when the additive solution is transferred from the second bag to the first bag using a height difference between the first bag and the second bag, air from the second bag stops in the middle of the flow path; and a hanging step of hanging the second bag such that a influx portion of the second bag faces downward.

The above-described method for transferring the additive solution may also be configured such that the support step hangs the first bag such that the influx portion of the first bag faces downward at a position lower than the second bag hung at the support step.

The above-described method for transferring the additive solution may also be configured such that the support step supports the first bag diagonally with respect to the horizontal direction, to arrange the first bag to be gradually lowered toward the influx portion of the first bag, at a position lower than the second bag to be hung.

The above-described method for transferring the additive solution may be configured such that the transfer line includes a first bag-side tube formed of a flexible material connected to the first bag, a second bag-side tube formed of a flexible material connected to the second bag, and a branch section formed to be harder than the first bag-side tube and the second bag-side tube and provided between the first bag-side tube and the second bag-side tube, and such that the support step arranges the liquid level inside the first bag to come at a position lower than the branch section.

The above-described method for transferring the additive solution may be also configured such that the support step supports the first bag and the transfer line so as to form, in the transfer line, a high-level site existing at a position higher than the liquid level inside the first bag, and a low-level site existing at a position lower than the high-level site on both sides of the flow path of the high-level site.

The above-described method for transferring the additive solution may be configured such that the transfer line includes a first bag-side tube formed of a flexible material connected to the first bag, a second bag-side tube formed of a flexible material connected to the second bag, and a branch section formed to be harder than the first bag-side tube and the second bag-side tube and provided between the first bag-side tube and the second bag-side tube, and such that the support step arranges the high-level site to come at a position lower than the branch section.

In the above-described method for transferring the additive solution, the blood component may be a concentrated red blood cell and the additive solution may be a red blood cell preservation. With this, it is possible to simplify the operation procedure at production of red blood cell products. Accordingly, it is possible to efficiently produce red blood cell products that conform to a predetermined quality standard.

With the hanging device and the method for transferring the additive solution according to the present invention, it is possible to efficientlyproduce blood products at a production process of the blood products by eliminating the need for manual air bleeding operation after addition of the additive solution.

### Brief Description of Drawings

Fig. 1 is a general schematic diagram of a blood bag system.
Fig. 2 is a perspective view of a centrifugation and separation apparatus.
Fig. 3 is a schematic diagram illustrating a state of the blood bag system before a centrifugation step.
Fig. 4 is a schematic diagram illustrating a state of the blood bag system after an air separation step and before a separation and separation step.
Fig. 5 is a schematic diagram illustrating a hanging device and a blood bag system set on the hanging device, according a first embodiment of the present invention.
Fig. 6 is a schematic diagram illustrating a hanging device and a blood bag system set on the hanging device, according a second embodiment of the present invention.
Fig. 7 is a schematic diagram illustrating a hanging device and a blood bag system set on the hanging device, according a third embodiment of the present invention.
Fig. 8 is a schematic diagram illustrating a hanging device according the third embodiment of the present invention, and illustrating a blood bag system set on the hanging device in a state different from the case in Fig. 7.

### Description of Embodiments

Hereinafter, a hanging device and a method for transferring an additive solution will now be described with preferred embodiments and attached drawings.

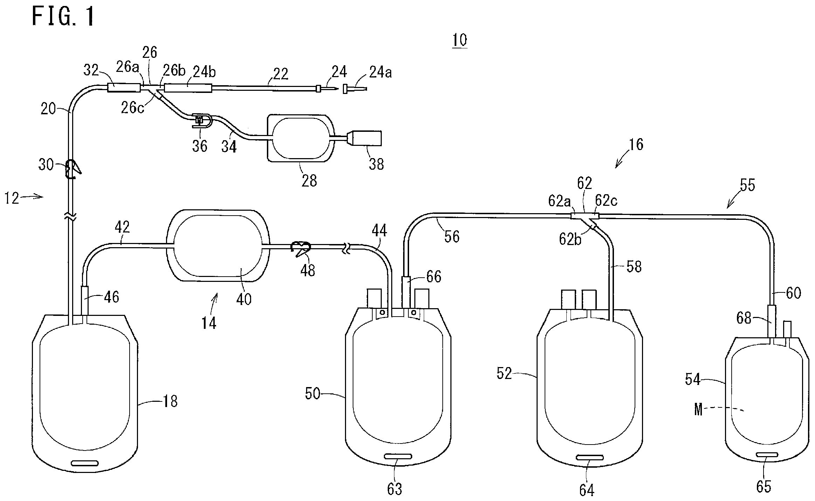

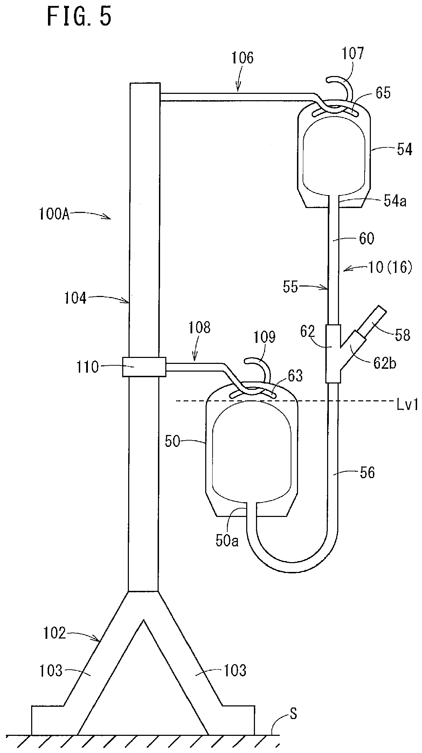

Before describing configurations of hanging devices 100A to 100C (refer to Figs. 5 to 8) according to embodiments of the present invention, a blood bag system 10 illustrated in Fig. 1 will be described. The blood bag system 10 is intended to centrifuge blood containing a plurality of components into a plurality of components having a different specific gravity (for example, two components including a relatively low specific gravity component and a relatively high specific gravity component) and to store and preserve the components separately in different bags.

The blood bag system 10 according to the present embodiment is configured to centrifuge a residual blood component obtained by removing white blood cells and platelets from whole blood into two components, namely, plasma and concentrated red blood cells, and to store and preserve the plasma and the concentrated red blood cells separately in different bags. The hanging devices 100A to 100C illustrated in Figs. 5 to 8 are used to hang a portion of the blood bag system 10 (separation processing unit 16 to be described below).

As illustrated in Fig. 1, the blood bag system 10 includes a blood collection unit 12, a preprocessing unit 14, and a separation processing unit 16. The blood collection unit 12 collects blood (whole blood) from a donor. The preprocessing unit 14 removes a predetermined blood component from the whole blood. The separation processing unit 16 centrifuges the residual blood component obtained by removing the predetermined component into a plurality of blood components and stores (pools) the individual blood components in different bags.

The blood collection unit 12 will first be described. The blood collection unit 12 includes a blood collection bag 18, blood collection tubes 20 and 22, a blood collection needle 24, a branching connector 26, and an initial flow blood bag 28.

The blood collection bag 18 is a bag for storing (pooling) blood (whole blood) collected from a donor. Preferably, an anticoagulant is placed in the blood collection bag 18 in advance . The anticoagulant is typically a liquid and may include, for example, an ACD-A solution, a CPD solution, a CPDA-1 solution, a heparin sodium solution, or the like. The amount of the anticoagulant is appropriately determined depending on the amount of blood to be collected.

The blood collection bag 18 is formed in a bag shape by overlapping flexible sheet materials formed of flexible resin such as polyvinyl chloride or polyolefin on each other and by fusion bonding (through thermal fusion bonding or high-frequency welding) or gluing on peripheral sealing portions of the sheet materials to each other. Similarly, the initial flow blood bag 28 is also formed in a bag shape.

One end of the proximal-side blood collection tube 20 is connected to the blood collection bag 18. A clamp 30 for closing or opening a flow path in the blood collection tube 20 is provided in the middle of the blood collection tube 20. One end of a frangible part 32 is connected to the other end of the blood collection tube 20. The frangible part 32 is configured such that the flow path is closed in an initial state and is then opened by performing breaking operation.

A first port 26a of the branching connector 26 is connected to the other end of the frangible part 32. One end of the distal-side blood collection tube 22 is connected to a second port 26b of the branching connector 26, and the blood collection needle 24 is connected to the other end of the blood collection tube 22. A cap 24a is attached to the blood collection needle 24 before use, and a needle guard 24b is attached to the blood collection needle 24 after use. The needle guard 24b is movably arranged along the longitudinal direction of the blood collection tube 22.

One end of a branching tube 34 is connected to a third port 26c of the branching connector 26. A clamp 36 that closes or opens a flow path of the branching tube 34 is provided in the middle of the branching tube 34. The initial flow blood bag 28 is connected to the other end of the branching tube 34. A sampling port 38 is connected to the initial flow blood bag 28. The direction and arrangement of the branching connector 26 are not limited to the configuration illustrated in Fig. 1 but may be appropriately changed.

The preprocessing unit 14 includes a filter 40, an inlet-side tube 42, and an outlet-side tube 44. The filter 40 removes predetermined cells. The inlet-side tube 42 is connected at its one end to the blood collection bag 18 and connected at its other end to an inlet of the filter 40. The outlet-side tube 44 is connected at its one end to an outlet of the filter 40 and connected at its other end to the separation processing unit 16.

In the present embodiment, the filter 40 is configured as a white blood cell removing filter. As the white blood cell removing filter, it is appropriate to use a filter having a structure that stores a liquid-passing porous body having numerous micropores communicating from one side surface to the other side surface thereof in a bag-shaped housing formed of a flexible resin sheet. In the present embodiment, the filter 40 is configured to be able to supplement platelets, as well.

The inlet-side tube 42 is a tube for transferreing the blood collected from a donor from the blood collection bag 18 to the filter 40, and is connected to a top portion of the blood collection bag 18. In the present embodiment, a frangible part 46 is provided at a blood collection bag 18-side end of the inlet-side tube 42. The frangible part 46 has the same configuration and function as those of the frangible part 32 described above.

The outlet-side tube 44 is a tube for transferreing the residual blood components obtained by removing predetermined cells (in the present embodiment, white blood cells and platelets) using the filter 40, to the separation processing unit 16 (specifically, a primary bag 50 described below). A clamp 48 that closes and opens a flow path of the outlet-side tube 44 is provided in the middle of the outlet-side tube 44. A clamp similar to the clamp 30 described above may be employed as the clamp 48.

Next, the separation processing unit 16 will be described. The separation processing unit 16 includes a primary bag (first bag) 50, a subsidiary bag (third bag) 52, a medical fluid bag (second bag) 54, and a transfer line 55. The primary bag (first bag) 50 stores (pools) the residual blood component obtained by removing the predetermined cells using the filter 40. The subsidiary bag (third bag) 52 stores and preserves a supernatant component obtained by centrifuging the blood component inside the primary bag 50. The medical fluid bag (second bag) 54 stores a red blood cell preservation M (additive solution). The transfer line 55 is connected to the primary bag 50, the subsidiary bag 52, and the medical fluid bag 54.

Similarly to the blood collection bag 18, the primary bag 50, the subsidiary bag 52 and the medical fluid bag 54 are each formed in a bag shape by overlapping flexible sheet materials formed of flexible resin such as polyvinyl chloride or polyolefin on each other and by fusion bonding (through thermal fusion bonding or high-frequency welding) or gluing on peripheral sealing portions of the sheet materials to each other. Slits 63 to 65 are provided on bottom portions of the primary bag 50, the subsidiary bag 52, and the medical fluid bag 54 so as to enable hanging each of the bags with their top portions facing downward.

The primary bag 50 serves as both a bag for storing (pooling) the residual blood component obtained by removing the predetermined cells using the filter 40 and a bag for preserving a sedimentary component (concentrated red blood cells) obtained by centrifuging the blood component.

The transfer line 55 is a means to connect the primary bag 50 with the subsidiary bag 52 and to connect the primary bag 50 with the medical fluid bag 54. In the exemplary illustration, the transfer line 55 includes a first tube 56 (first bag-side tube) connected to the primary bag 50, a second tube 58 connected to the subsidiary bag 52, a third tube 60 (second bag-side tube) connected to the medical fluid bag 54, and a branching connector 62 (branch section) connected to the first to third tubes 56, 58, and 60.

A breakable frangible part 66 is provided at one end (primary bag 50-side end) of the first tube 56 to prevent the blood component inside the primary bag 50 from being transferred to other bags until the breaking operation is performed. The frangible part 66 has the same configuration and functionality as those of the frangible part 32 described above.

The other end of the first tube 56 is connected to the first port 62a of the branching connector 62. One end of the second tube 58 is connected to the second port 62b of the branching connector 62. One end of the third tube 60 is connected to the third port 62c of the branching connector 62. The branching connector 62 is formed of a material harder than the material of the first to third tubes 56, 58, and 60.

As the red blood cell preservation M contained in the medical fluid bag 54, a MAP solution, an SAGM solution, OPTISOL, or the like, may be employed. A breakable frangible part 68 is provided at the medical fluid bag 54-side end of the third tube 60 to prevent the red blood cell preservation liquid M in the medical fluid bag 54 from being transferred to other bags. The frangible part 68 has the same configuration and functionality as those of the frangible part 32 described above.

The tubes in the blood bag system 10 are formed of transparent flexible resin. The clamps 30, 36 and 48 may be standard products that have been conventionally used. The clamps 30, 36, and 48 are preferably classified by different colors depending on a place or purpose of use. When the blood bag system 10 is sterilized or stored before use, the clamps 30, 36, and 48 are in open states, so that the bags internally communicate with each other to maintain a uniformly sterilized state.

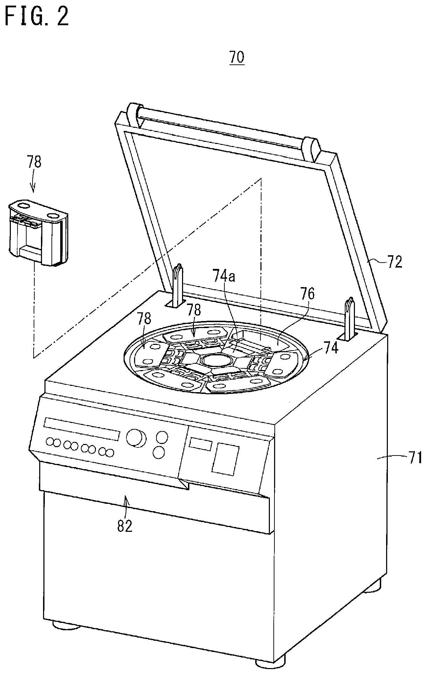

The blood bag system 10 may be, for example, attached to a centrifugation and separation apparatus 70 illustrated in Fig. 2 in use. The centrifugation and separation apparatus 70 is used to centrifuge the blood component stored inside the primary bag 50 into two layers, namely, a plasma layer and a concentrated red blood cell layer, and deliver the plasma into the subsidiary bag 52 while keeping the concentrated red blood cells to remain inside the primary bag 50.

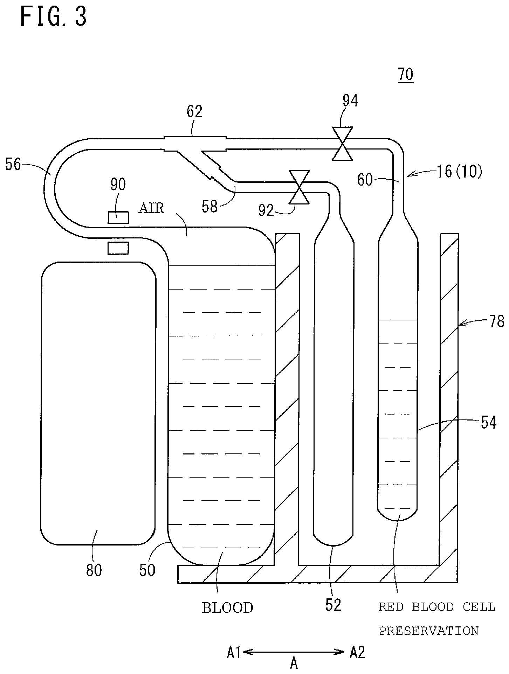

The centrifugation and separation apparatus 70 has a box shape and includes a main body 71, a lid 72, a centrifugal drum 74, six unit insertion holes 76, six insert units 78, and six pressers 80 (refer to Fig. 3, etc.). The lid 72 is openable and closable on an upper surface. The centrifugal drum 74 is internally installed. The six unit insertion holes 76 are arranged inside the centrifugal drum 74 at a regular angular interval (60°). The six insert units 78 are insertable and removable respectively into/from the unit insertion holes 76. The six pressers 80 provided at a center, and are capable of advancing and retreating in a rotational radial direction with respect to each of the insert units 78. The centrifugation and separation apparatus 70 is operated based on operation of an operation unit 82 provided in front and is controlled by a microcomputer (not illustrated).

Typically six insert units 78 are attached to the centrifugation and separation apparatus 70. Alternatively, it is also allowable to attach five or less insert units (preferably, three or two insert units arranged at a regular angular interval) as long as the insert units are appropriately balanced.

Before the blood bag system 10 is attached to the centrifugation and separation apparatus 70 after the residual blood component obtained by filtering whole blood using the filter 40 is transferred to the primary bag 50 and stored therein, the outlet-side tube 44 is fusion-bonded to prevent leakage using a tube sealer, or the like, and is then cut. Accordingly, among the blood bag system 10, merely the separation processing unit 16 and a portion of the outlet-side tube 44 are attached to the insert unit 78.

Processing including collecting blood from a donor using the blood bag system 10 illustrated in Fig. 1, removing white blood cells and platelets from collected blood, separating residual components into two layers of plasma and concentrated red blood cells, and then pooling the separated components in the separate bags can be performed by the following procedure, for example.

First, using the blood collection needle 24, blood collection step is performed such that the skin of a donor is punctured and the blood is collected from the donor. In the blood collection step, a predetermined amount of initial flow of the blood (initial-flow-at-blood-collection) collected from a donor is stored in the initial flow blood bag 28 before the blood is collected into the blood collection bag 18. In this case, the clamp 36 is set to an open state while the frangible part 32 is kept to a closed state (initial state) . As a result, it is possible to block the entry of initial-flow-at-blood-collection into the blood collection tube 20 side, namely, the blood collection bag 18 side, and also possible to introduce the initial-flow-at-blood-collection into the initial flow blood bag 28 via the blood collection tube 22, the branching connector 26, and the branching tube 34.

Next, by attaching a blood sampling tube (not illustrated) to the sampling port 38, initial-flow-at-blood-collection is collected into the blood sampling tube. The initial-flow-at-blood-collection is used as blood sample for testing. Portions from the branching connector 26 to the sampling port 38 may be omitted depending on application.

After collection of the initial-flow-at-blood-collection is completed, the branching tube 34 is closed by the clamp 36, and breaking operation is performed for the frangible part 32 to open the flow path of the blood collection tube 20. At this time, when the clamp 30 is set to an open state, the blood from the donor flows sequentially through the blood collection tubes 22 and 20 into the blood collection bag 18.

After a predetermined amount of blood is collected and pooled in the blood collection bag 18, the blood collection tube 20 is closed by the clamp 30 so as not to let the blood in the blood collection bag 18 flow out. After the blood collection tube 20 is fusion-bonded and sealed by a tube sealer, or the like, the blood collection tube 20 is then cut at the sealed portion.

Then, with the blood collection bag 18 being arranged in an upper position and the primary bag 50 being arranged in a lower position, the filter 40 is arranged in an intermediate position. Thereafter, breaking operation is performed on the frangible part 46 provided at one end portion of the inlet-side tube 42 to open the flow path of the inlet-side tube 42. As a result, the whole blood in the blood collection bag 18 flows through the inlet-side tube 42 into the filter 40, and with white blood cells and platelets removed in the course of passing through the filter 40, flows through the outlet-side tube 44 into the primary bag 50 to be collected therein. At this time, not only the blood from which white blood cells and platelets have been removed but also air existing in the filter 40 flows into the primary bag 50.

Then, the outlet-side tube 44 is fusion-bonded and sealed at a position downstream of the clamp 48 using a tube sealer, or the like, and the outlet-side tube 44 is cut at the sealed portion.

Then, to separate the blood component collected in the primary bag 50 into plasma and concentrated red blood cells and pool them in predetermined bags individually, the separation processing unit 16 of the blood bag system 10 is attached to the centrifugation and separation apparatus 70. At this time of attaching, the above-described breaking operation is performed on the frangible part 66 so as to open the flow path of the frangible part 66. The breaking operation of the frangible part 66 may be performed before or after attaching the primary bag 50 to the centrifugation and separation apparatus 70.

Subsequently, the blood bag system 10 (specifically, separation processing unit 16) is attached to the insert unit 78, and the insert unit 78 is then inserted into the unit insertion hole 76 to achieve the state of the schematic diagram illustrated in Fig. 3. In Fig. 3, the directions A indicated by the double arrow represent radial directions of the centrifugal drum 74 of the centrifugation and separation apparatus 70. More specifically, the direction A1 represents an inward radial direction and the direction A2 represents an outward radial direction.

The primary bag 50, while being attached to the insert unit 78, is held vertically (extending in an up-down direction), and stored in the centrifugation and separation apparatus 70 such that the thickness direction of the bag main body is arranged in the directions A. With this arrangement, during rotation of the centrifugal drum 74 of the centrifugation and separation apparatus 70, centrifugal force is applied to the primary bag 50 in the A2 direction.

As illustrated in Fig. 3, the centrifugation and separation apparatus 70 includes a sensor 90 that detects the type of liquid passing through the first tube 56. The sensor 90 includes, for example, a light-transmitting section and a light-receiving section and can determine the type of the liquid passing there between based on a light transmission level of the liquid. The centrifugation and separation apparatus 70 further includes a clamp 92 and a clamp 94. The clamp 92 is operable to open and close and switches open/close of the flow path of the second tube 58 by open/close operation. The clamp 94 is operable to open and close and switches open/close of the flow path of the third tube 60 by open/close operation.

Alternatively, it is also allowable to configure such that the clamps 92 and 94 are attached to the second tube 58 and the third tube 60 respectively beforehand and that an operation mechanism to open and close the clamps 92 and 94 is provided in the centrifugation and separation apparatus 70.

Next, the lid 72 of the centrifugation and separation apparatus 70 is closed, and thereafter, the centrifugation step and the separation-transfer step are automatically performed by operating the operation unit 82. As illustrated in Fig. 3, the clamps 92 and 94 are closed before the centrifugation step is started. Accordingly, flow paths of the second and third tubes 58 and 60 are closed. Air exists in a top portion inside the primary bag 50. This air is the air that existed in the filter 40 and flew into the primary bag 50 when blood filtration is performed by the filter 40.

First, in automatic operation of the centrifugation and separation apparatus 70, the centrifugation step is performed by rotating the centrifugal drum 74, that is, the blood component pooled in the primary bag 50 is separated into plasma and concentrated red blood cells. In the centrifugation step, the blood component pooled in the primary bag 50 receives a centrifugal force. As a result, the concentrated red blood cells as a high specific gravity component are moved in the outward radial direction (direction A2), whereas the plasma as a low specific gravity component is moved in the inward radial direction (direction A1), and thus, the blood component is separated into two layers.

The centrifugation and separation apparatus 70 performs an air separation step after the centrifugation step. In the air separation step, the clamp 94 is opened while the rotation of centrifugal drum 74 is maintained, thereby switching the flow path of the third tube 60 to an open state.

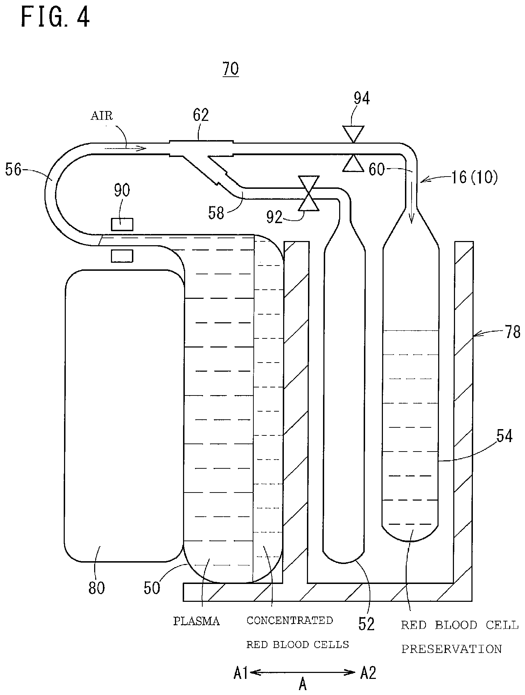

Next, as illustrated in Fig. 4, the primary bag 50 is pressed by displacing the presser 80 in the outward radial direction (direction A2). Since the volume of the primary bag 50 is reduced because it is clamped between the presser 80 and the wall, air is discharged from inside the primary bag 50. Accordingly, the air is flown into the medical fluid bag 54 via the first tube 56, the branching connector 62, and the third tube 60.

The centrifugation and separation apparatus 70 performs plasma separation and separation step after the air separation step. Specifically, after the air has flown out of the primary bag 50, since the first tube 56 is directed toward the inner diameter side, the plasma located in the innermost diameter side starts to flow out of the primary bag 50. At this time, when the flow of the plasma into the first tube 56 is detected by the sensor 90, the third tube 60 is switched to a closed state by closing the clamp 94 while maintaining the rotation of the centrifugal drum 74, and together with this, the second tube 58 is switched to an open state by opening the clamp 92. This allows the plasma that has flown out of the primary bag 50 to flow into the subsidiary bag 52 via the first tube 56, the branching connector 62, and the second tube 58.

After the plasma has completely flown out of the primary bag 50, the concentrated red blood cells start to flow out of the primary bag 50. At this time, when the flow of the red blood cells into the first tube 56 is detected by the sensor 90, the presser 80 is stopped, and the flow path of the second tube 58 is closed by closing the clamp 92. Accordingly, it is possible to block the entry of flow of the red blood cells into the subsidiary bag 52.

After completion of the above-described separation-transfer step, the separation processing unit 16 is taken out of the insert unit 78 and the second tube 58 is fusion-bonded and sealed by a tube sealer, or the like, and thereafter, is cut, thereby separating the subsidiary bag 52.

Welding, sealing and cutting of the second tube 58 using the tube sealer, or the like, may also be performed within the centrifugation and separation apparatus 70 using mechanical operation, by adding a tube-sealing function, cutting function, or the like, to the clamp 92.

Next, the addition step is performed manually. In this step, the red blood cell preservation liquid M is added to the concentrated red blood cells. The addition step can be performed, for example, by using the hanging device 100A according to the first embodiment, illustrated in Fig. 5. The hanging device 100A is used when the red blood cell preservation liquid M is transferred from the medical fluid bag 54 to the primary bag 50 using the height difference between the primary bag 50 and the medical fluid bag 54, in order to add the red blood cell preservation liquid M to the concentrated red blood cells inside the primary bag 50.

The hanging device 100A includes a base 102, a support column 104, and two hanging sections 106 and 108 (second bag hanging section, support mechanism). The base 102 is placed on an installation surface S. The support column 104 is supported by the base 102 and extends in the up-down direction . The hanging sections 106 and 108 are supported by the support column 104 with an interval in the up-down direction. The base 102 has a plurality of legs 103 in an exemplary illustration. The form, however, is not limited to this, but may have a plate shape expanding in the horizontal direction.

Among the two hanging sections 106 and 108, the hanging section 106 provided relatively at an upper side (hereinafter, referred to as an "upper hanging section 106") is configured to be able to hang the medical fluid bag 54 such that a influx portion 54a of the medical fluid bag 54 faces downward (namely, upside down). Specifically, the upper hanging section 106 is formed into a stick-like shape protruding in a direction (horizontal direction in the exemplary illustration) crossing the support column 104. A curved hook section 107 is provided at a free end-side of the upper hanging section 106. By inserting the hook section 107 into the slit 65 provided on a bottom portion of the medical fluid bag 54, it is possible to hang the medical fluid bag 54 upside down.

Among the two hanging sections 106 and 108, the hanging section 108 provided relatively at lower side (hereinafter, referred to as an "lower hanging section 108") is configured to be able to hang the primary bag 50 such that a influx portion 50a of the primary bag 50 faces downward (namely, upside down) at a position lower than the medical fluid bag 54 hung by the upper hanging section 106.

Specifically, the lower hanging section 108 is formed into a stick-like shape protruding in a direction (horizontal direction in the exemplary illustration) crossing the support column 104. A curved hook section 109 is provided at a free end-side of the lower hanging section 108. By inserting the hook section 109 into the slit 63 provided on a bottom portion of the primary bag 50, it is possible to hang the primary bag 50 upside down.

The lower hanging section 108 configured in this manner functions as a support mechanism to support the primary bag 50 in a predetermined state such that, when the red blood cell preservation liquid M is transferred from the medical fluid bag 54 to the primary bag 50 using the height difference between the primary bag 50 and the medical fluid bag 54, air from the medical fluid bag 54 stops in the middle of the transfer line 55.

As an alternative means of hanging the medical fluid bag 54 or the primary bag 50 upside down besides the hook sections 107 and 109, it is allowable to employ, for example, a clip having a pair of gripping sections capable of providing a gripping force given by an elastic force of a spring. By gripping and holding the bottom portion of the medical fluid bag 54 or the primary bag 50, it is possible to hang the medical fluid bag 54 or the primary bag 50 upside down.

In Fig. 5, the upper hanging section 106 and the lower hanging section 108 have difference lengths. The lengths, however, may be the same.

As illustrated in Fig. 5, the hanging device 100A may further include a height adjustment mechanism 110 for adjusting the hanging height when hung by the lower hanging section 108. In an exemplary illustration, the height adjustment mechanism 110 is slidable in the up-down direction with respect to the support column 104 and can hold an adjusted height position. As modification of the height adjustment mechanism 110, it is allowable to employ a configuration that can adjust the hanging height using the lower hanging section 108 that can change its angle with respect to the support column 104.

In a case where the addition step is performed using the above-configured hanging device 100A, the blood bag system 10 (separation processing unit 16) is set on the hanging device 100A as illustrated in Fig. 5. Specifically, the primary bag 50 is hung by hooking the primary bag 50 at a position of the slit 63 onto a hook section 109 of the lower hanging section 108 such that the influx portion 50a of the primary bag 50 faces downward. Next, the medical fluid bag 54 is hung by hooking the medical fluid bag 54 at a position of the slit 65 onto the hook section 107 of the upper hanging section 106 such that the influx portion 54a of the medical fluid bag 54 faces downward.

In this case, in order to facilitate squeezing operation to be described below, it is preferable to arrange a liquid level Lv1 inside the primary bag 50 to be at a position lower than the branching connector 62, as illustrated in Fig. 5. In the hanging device 100A, the hanging height of the primary bag 50 is adjustable with the height adjustment mechanism 110, and thus, it is possible to easily set the liquid level Lv1 inside the primary bag 50 to a position lower than the branching connector 62.

In a case where it is difficult to provide the height adjustment mechanism 110 in the hanging device 100A, it would be preferable that the position of the lower hanging section 108 has been set beforehand such that the liquid level Lv1 inside the primary bag 50 comes at a position lower than the branching connector 62 with the medical fluid bag 54 and the primary bag 50 being respectively hung on the upper hanging section 106 and the lower hanging section 108.

In this manner, when the medical fluid bag 54 containing the red blood cell preservation liquid M is arranged upside down at a relatively high position, and when the primary bag 50 containing the concentrated red blood cells is arranged upside down at a relatively low position, the red blood cell preservation liquid M inside the medical fluid bag 54 starts to move downward by gravity due to the height difference between the medical fluid bag 54 and the primary bag 50. In this case, air exists in a top portion inside the medical fluid bag 54, namely, in a portion above the red blood cell preservation liquid M.

As a result, after flowing of the red blood cell preservation liquid M out of the medical fluid bag 54 is finished, air existing inside the medical fluid bag 54 starts to flow toward the third tube 60. In this case, the air moving inside the transfer line 55 from the medical fluid bag 54 stops at a level corresponding to a liquid level Lv1 (or around this level) inside the primary bag 50. In other words, the liquid level Lv1 inside the primary bag 50 hung upside down is at a height in the middle of the transfer line 55. Accordingly, since the liquid level Lv1 (or around this level) can be balanced with the liquid level of the red blood cell preservation liquid M inside the transfer line 55, movement of air stops when the movement of the red blood cell preservation liquid M stops. As a result, the air from the medical fluid bag 54 is least likely to flow into the primary bag 50.

Meanwhile, at a stage before starting the addition step, the third tube 60 includes air inside itself and this air flows into the primary bag 50 at an initial stage of the addition step. Fortunately, however, the amount of air inside the third tube 60 is significantly less than the amount of air inside the medical fluid bag 54, and thus, the amount of air flowing into the primary bag 50 at the addition step would be small. Accordingly, after completion of the addition step, the amount of air inside the primary bag 50 is the amount that scarcely needs air bleeding operation.

When the movement of the red blood cell preservation liquid M and the movement of the air have stopped, operation to move the red blood cell preservation liquid M remaining in the flow path of the transfer line 55 to the primary bag 50 (squeezing operation) is performed by squeezing the transfer line 55 toward the primary bag 50 side. In this exemplary case, however, unlike the state in Fig. 5, if the liquid level Lv1 inside the primary bag 50 is at the height of the branching connector 62 or above, the air from the medical fluid bag 54 would stop at a position at the height of the branching connector 62 or above. This would make works complicated not only because the branching connector 62 hinders squeezing operation but also because the first tube 56 has to be sealed and cut after the third tube 60 has been sealed and cut.

In contrast, in a case where the liquid level Lv1 inside the primary bag 50 is at a position lower than the branching connector 62 as in Fig. 5, it is sufficient to squeeze the first tube 56 alone, and since the branching connector 62 would not hinder operation, it is possible to smoothly perform squeezing operation. Furthermore, it is sufficient that the first tube 56 alone is sealed and cut, and thus, the work would be simple.

With the above-described processing, it is possible to remove white blood cells and platelets from whole blood, separate residual blood components into two components, namely, plasma and concentrated red blood cells, and to store and preserve the plasma and the concentrated red blood cells separately in different bags (primary bag 50 and the subsidiary bag 52).

As described above, with the hanging device 100A according to the present embodiment, the air from the medical fluid bag 54 stops in the middle of the transfer line 55 when the red blood cell preservation liquid M is transferred from the medical fluid bag 54 to the primary bag 50 using the height difference between the primary bag 50 and the medical fluid bag 54 in order to add the red blood cell preservation liquid M to the concentrated red blood cells inside the primary bag 50. This can prevent the air from the medical fluid bag 54 from mixing into the primary bag 50. Accordingly, after the red blood cell preservation liquid M has been added to the concentrated red blood cells, the amount of air inside the primary bag 50 is the amount that scarcely needs air bleeding operation. As a result, it is possible to eliminate air bleeding operation after addition of the red blood cell preservation liquid M.

According to the present embodiment, by hanging the primary bag 50 upside down, the liquid level Lv1 inside the primary bag 50 comes at a position higher than the influx portion 50a of the primary bag 50 when the additive solution is transferred from the medical fluid bag 54 to the primary bag 50. Accordingly, the air moving inside the transfer line 55 from the medical fluid bag 54 stops at a position of the liquid level Lv1 inside the primary bag 50. As a result, it is possible, with a simple configuration, to appropriately prevent the air from the medical fluid bag 54 from mixing into the primary bag 50.

The above-described hanging device 100A is configured to arrange both the upper hanging section 106 and the lower hanging section 108 to be on the same stand (base 102 and support column 104). Alternatively, the upper hanging section 106 and the lower hanging section 108 may be provided on separate stands or constructions.

In the above-described addition step, the primary bag 50 may be hung upside down by hanging the primary bag 50 at the slit 63 on a second port 62b of the branching connector 62 instead of hanging the primary bag 50 upside down on the lower hanging section 108. Even with this method, the air moving inside the transfer line 55 from the medical fluid bag 54 stops at a level corresponding to a liquid level Lv1 inside the primary bag 50. Accordingly, similarly to the addition step in Fig. 5, it is possible to appropriately prevent the air from the medical fluid bag 54 from mixing into the primary bag 50. Furthermore, it is also possible to configure to provide a protruding portion (e.g., another port) capable of hanging the primary bag 50 at the position of the slit 63, on the branching connector 62, and hang the primary bag 50 upside down on the protruding portion. In a case where the primary bag 50 is hung on the branching connector 62, the lower hanging section 108 can be omitted.

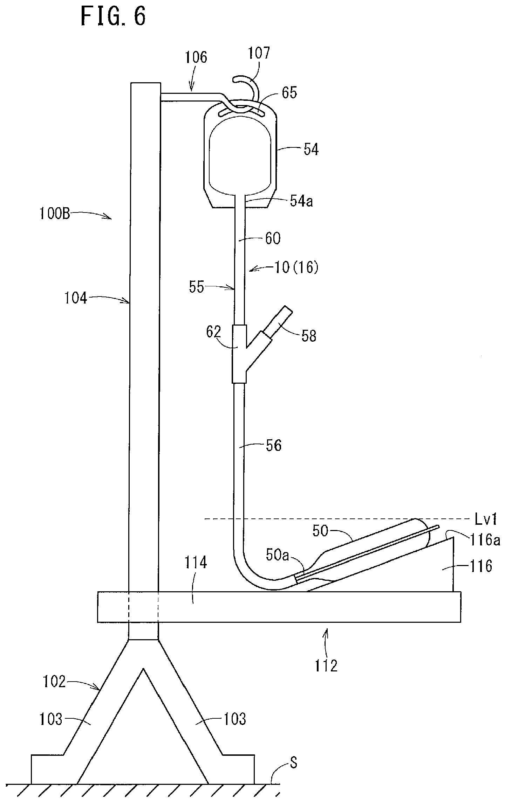

The above-described addition step can be performed by using a hanging device 100B according to a second embodiment illustrated in Fig. 6. On the hanging device 100B, constituent elements shared with the hanging device 100A have the same reference signs . The hanging device 100B includes a bag mounting section 112 for mounting the primary bag 50, instead of the lower hanging section 108 on the hanging device 100A described above.

The bag mounting section 112 is provided below the upper hanging section 106 and capable of supporting the primary bag 50 diagonally with respect to the horizontal direction to arrange the primary bag 50 to be gradually lowered toward the influx portion 50a side of the primary bag 50, at a position lower than the medical fluid bag 54 hung by the upper hanging section 106.

As illustrated in Fig. 6, the bag mounting section 112 includes, for example, a platform 114 and an inclined table 116. The platform 114 is supported by the support column 104 and extends in the horizontal direction. The inclined table 116 is provided on the platform 114. By mounting the primary bag 50 on a diagonal mounting surface 116a forming an upper surface of the inclined table 116 with the influx portion 50a coming on a lower side, it is possible to support the primary bag 50 diagonally while keeping the state where the influx portion 50a of the primary bag 50 is at a relatively lower position and a bottom portion of the primary bag 50 is at a relatively higher position.

It is preferable to set the relative positional relationship between the bag mounting section 112 and the upper hanging section 106 such that the liquid level Lv1 inside the primary bag 50 comes at a position lower than the branching connector 62 in a state where the medical fluid bag 54 is hung by the upper hanging section 106 and the primary bag 50 is mounted on the bag mounting section 112. The bag mounting section 112 may be configured to be height-adjustable with respect to the support column 104.

The bag mounting section 112 configured in this manner functions as a support mechanism to support the primary bag 50 in a predetermined state such that air from the medical fluid bag 54 stops in the middle of the transfer line 55 when the red blood cell preservation liquid M is transferred from the medical fluid bag 54 to the primary bag 50 using the height difference between the primary bag 50 and the medical fluid bag 54.

In a case where the addition step is performed using the above-configured hanging device 100B, the blood bag system 10 (separation processing unit 16) is mounted on the hanging device 100B as illustrated in Fig. 6. Specifically, the primary bag 50 is supported diagonally while keeping the state where the influx portion 50a of the primary bag 50 is at a relatively lower position and the liquid level Lv1 inside the primary bag 50 is at a relatively higher position by mounting the primary bag 50 on the inclined table 116 of the bag mounting section 112 with the influx portion 50a coming on a lower side. Next, the medical fluid bag 54 is hung by hooking the medical fluid bag 54 at a position of the slit 65 onto the hook section 107 of the upper hanging section 106 such that the influx portion 54a of the medical fluid bag 54 faces downward. As a result, the liquid level Lv1 inside the primary bag 50 comes at a position lower than the branching connector 62.

In this manner, when the medical fluid bag 54 containing the red blood cell preservation liquid M is arranged upside down at a relatively high position, and when the primary bag 50 containing the concentrated red blood cells is arranged at a relatively low position, the red blood cell preservation liquid M in the medical fluid bag 54 starts to move downward by gravity due to the height difference between the medical fluid bag 54 and the primary bag 50. In this case, air exists in a top portion inside the medical fluid bag 54, namely, in a portion above the red blood cell preservation liquid M.

As a result, after flowing of the red blood cell preservation liquid M out of the medical fluid bag 54 is finished, air existing inside the medical fluid bag 54 starts to flow toward the third tube 60. In this case, the air moving inside the transfer line 55 from the medical fluid bag 54 stops at a level corresponding to a liquid level Lv1 (or around this level) inside the primary bag 50. As a result, the air from the medical fluid bag 54 is least likely to flow into the primary bag 50.

When the movement of the red blood cell preservation liquid M and movement of the air stop, squeezing operation is performed similarly to the case of the hanging device 100A. On the hanging device 100B, the red blood cell preservation liquid M remaining inside the transfer line 55 is at a position lower than the branching connector 62. Accordingly, it is sufficient to squeeze the first tube 56 alone. Accordingly, since the branching connector 62 does not hinder operation, it is possible to smoothly perform squeezing operation.

As described above, even with the hanging device 100B, it is possible to prevent the air from the medical fluid bag 54 from mixing into the primary bag 50. Accordingly, it is possible to eliminate air bleeding operation after addition of the red blood cell preservation liquid M.

On the hanging device 100B, by disposing the primary bag 50 diagonally with its influx portion 50a coming on the lower side, the liquid level inside the primary bag 50 comes at a position higher than the influx portion 50a of the primary bag 50 when the red blood cell preservation liquid M is transferred from the medical fluid bag 54 to the primary bag 50. Accordingly, the air moving inside the transfer line 55 from the medical fluid bag 54 stops at a position of the liquid level Lv1 inside the primary bag 50. As a result, it is possible, with a simple configuration, to appropriately prevent the air from the medical fluid bag 54 from mixing into the primary bag 50.

The above-described hanging device 100B is configured to arrange both the upper hanging section 106 and the bag mounting section 112 to be on the same stand (base 102 and support column 104). Alternatively, the upper hanging section 106 and the bag mounting section 112 may be provided on separate stands or constructions.

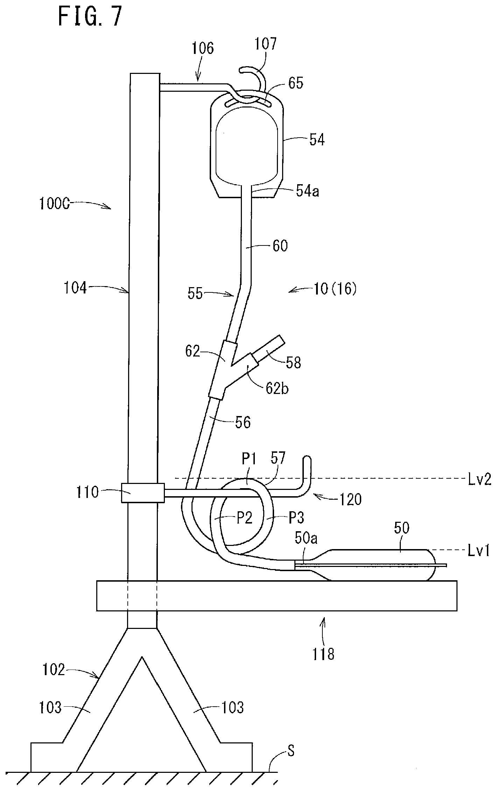

The above-described addition step can be performed by using a hanging device 100C according to a third embodiment illustrated in Fig. 7. On the hanging device 100C, constituent elements shared with the hanging device 100A have the same reference signs . The hanging device 100C includes a bag mounting section 118 for mounting the primary bag 50, and a tube supporting section 120 that supports a portion of the transfer line 55, instead of the above-described lower hanging section 108 in the hanging device 100A.

The bag mounting section 118 is provided below the upper hanging section 106 and capable of supporting the primary bag 50 horizontally at a position lower than the medical fluid bag 54 hung by the upper hanging section 106.

The tube supporting section 120 is supported by the support column 104 at a height between the upper hanging section 106 and the bag mounting section 118. The tube supporting section 120 supports the transfer line 55 so as to form a high-level site P1, and low-level sites P2, and P3 in the transfer line 55. The high-level site P1 is located at a position higher than the liquid level Lv1 inside the primary bag 50. The low-level sites P2 and P3 are located at positions lower than the high-level site P1 on both sides on the flow path of the high-level site P1. In Fig. 7, a loop 57 is formed on the first tube 56. The tube supporting section 120 supports the first tube 56 by allowing the tube supporting section 120 passing inside the loop 57.

In Fig. 7, the tube supporting section 120 is supported by the support column 104 via the height adjustment mechanism 110 similar to the height adjustment mechanism 110 illustrated in Fig. 5. The height of the tube supporting section 120 can be adjusted by the height adjustment mechanism 110.

It is preferable to set the relative positional relationship between the upper hanging section 106, the bag mounting section 118, and the tube supporting section 120 such that the high-level site P1 comes at a position lower than the branching connector 62 in a state where the medical fluid bag 54 is hung by the upper hanging section 106 and the primary bag 50 is mounted on the bag mounting section 118, and a portion of the transfer line 55 is supported by the tube supporting section 120.

Each of the bag mounting section 118 and the tube supporting section 120 configured as above functions as a support mechanism to support the primary bag 50 in a predetermined state such that air from the medical fluid bag 54 stops in the middle of the transfer line 55 when the red blood cell preservation liquid M is transferred from the medical fluid bag 54 to the primary bag 50 using the height difference between the primary bag 50 and the medical fluid bag 54.

In a case where the addition step is performed using the above-configured hanging device 100C, the blood bag system 10 (separation processing unit 16) is set on the hanging device 100C as illustrated in Fig. 7. Specifically, the primary bag 50 is mounted on its side on the bag mounting section 118. Next, the loop 57 is formed in the middle of the transfer line 55 (in an exemplary illustration, the first tube 56). Then, by hanging the loop 57 on the tube supporting section 120, the tube supporting section 120 supports the transfer line 55.

Next, the medical fluidbag 54 is hung by hooking the medical fluid bag 54 at a position of the slit 65 onto the hook section 107 of the upper hanging section 106 such that the influx portion 54a of the medical fluid bag 54 faces downward. As a result, the high-level site P1 is formed in the transfer line 55, at a position higher than the liquid level Lv1 inside the primary bag 50, and together with this, a liquid level Lv2 of the high-level site P1 comes at a position lower than the branching connector 62.

In this manner, when the medical fluid bag 54 containing the red blood cell preservation liquid M is arranged upside down at a relatively high position, and when the primary bag 50 containing the concentrated red blood cells is arranged at a relatively low position, the red blood cell preservation liquid M in the medical fluid bag 54 starts to move downward by gravity due to the height difference between the medical fluid bag 54 and the primary bag 50. In this case, air exists in a top portion inside the medical fluid bag 54, namely, in a portion above the red blood cell preservation liquid M.

As a result, after flowing of the red blood cell preservation liquid M out of the medical fluid bag 54 is finished, air existing inside the medical fluid bag 54 starts to flow toward the third tube 60. In this case, the air moving inside the transfer line 55 from the medical fluid bag 54 stops at a level corresponding to the liquid level Lv2 (or around this level) of the high-level site P1. As a result, the air from the medical fluid bag 54 is least likely to flow into the primary bag 50.

When the movement of the red blood cell preservation liquid M and movement of the air stop, squeezing operation is performed similarly to the case of the hanging device 100A. On the hanging device 100C, the red blood cell preservation liquid M remaining in the transfer line 55 is at a position lower than the branching connector 62. Accordingly, it is sufficient to squeeze the first tube 56 alone. Accordingly, since the branching connector 62 does not hinder operation, it is possible to smoothly perform squeezing operation.

As described above, even with the hanging device 100C, it is possible to prevent the air coming from the medical fluid bag 54 from mixing into the primary bag 50. Accordingly, it is possible to eliminate air bleeding operation after addition of the red blood cell preservation liquid M.

In addition, in the case where the hanging device 100C is applied, when the red blood cell preservation liquid M is transferred from the medical fluid bag 54 to the primary bag 50, the air moving inside the transfer line 55 from the medical fluid bag 54 stops at a position of the liquid level Lv2 (or around this level) of the high-level site P1. As a result, it is possible, with a simple configuration, to appropriately prevent the air from the medical fluid bag 54 from mixing into the primary bag 50.

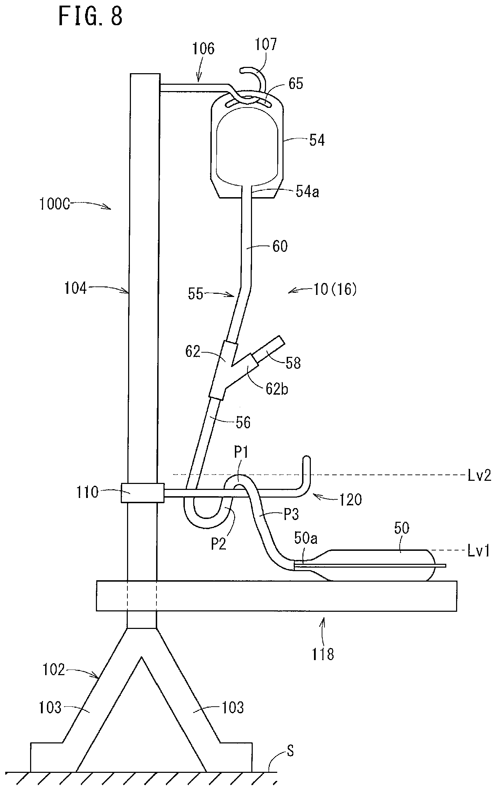

In a case where the addition step is performed using the hanging device 100C, the blood bag system 10 (separation processing unit 16) may be set as illustrated in Fig. 8. In this case, transfer line 55 is supported by the tube supporting section 120 with a portion in the middle of the transfer line 55 (in an exemplary illustration, the first tube 56) winding vertically. With this configuration, the high-level site P1, and the low-level sites P2, and P3 are formed in the transfer line 55. The high-level site P1 is located at a position higher than the liquid level Lv1 inside the primary bag 50. The low-level sites P2 and P3 are located at positions lower than the high-level site P1 on both sides on the flow path of the high-level site P1.

Even in a case of Fig. 8, that is, in a case where the addition step is performed while the blood bag system 10 (separation processing unit 16) is set on the hanging device 100C, the air moving inside the transfer line 55 from the medical fluid bag 54 stops at a position of the liquid level Lv2 (or around this level) of the high-level site P1. Accordingly, it is possible to prevent the air from the medical fluid bag 54 from mixing into the primary bag 50.

The above-described hanging device 100C is configured to arrange the upper hanging section 106, the bag mounting section 118, and the tube supporting section 120 to be on the same stand (base 102 and support column 104). Alternatively, the upper hanging section 106, the bag mounting section 118, and the tube supporting section 120 may be provided on separate stands or constructions.

In the addition step using the hanging device 100C, the portion in the middle of the transfer line 55 (first tube 56) may be hung on the second port 62b on the branching connector 62 instead of hanging the portion in the middle of the transfer line 55 (first tube 56) on the tube supporting section 120. With this configuration, the high-level site P1, and the low-level sites P2, and P3 are formed in the transfer line 55. The high-level site P1 is located at a position higher than the liquid level Lv1 inside the primary bag 50. The low-level sites P2 and P3 are located at positions lower than the high-level site P1 on both sides on the flow path of the high-level site P1. As a result, it is possible, similarly to the addition steps in Figs. 6 and 7, to appropriately prevent the air from the medical fluid bag 54 from mixing into the primary bag 50. Furthermore, it is also allowable to provide a protruding portion (e.g., another port) capable of hanging the portion in the middle of the transfer line 55 on the branching connector 62, and hang the portion in the middle of the transfer line 55 on the protruding portion. Note that in a case the portion in the middle of the transfer line 55 is hung on the branching connector 62, the tube supporting section 120 can be omitted.

In the above, preferred embodiments regarding the present invention have been described.

## Claims

1. A method for transferring an additive solution targeted at a blood bag system (10) including a first bag (50) containing a blood component, a second bag (54) containing additive solution (M), and a transfer line (55) that forms a flow path between the first bag (50) and the second bag (54), the method being configured to transfer the additive solution (M) from the second bag (54) to the first bag (50),
the method for transferring the additive solution comprising:
a support step of supporting the first bag (50) or the transfer line (55) in a predetermined state such that, when the additive solution (M) is transferred from the second bag (54) to the first bag (50) using a height difference between the first bag (50) and the second bag (54), air from the second bag (54) stops in the middle of the flow path; and
a hanging step of hanging the second bag (54) such that an influx portion (54a) of the second bag (54) faces downward,
**characterized in that** the support step supports the first bag (50) diagonally with respect to a horizontal direction to arrange the first bag (50) to be gradually lowered toward an influx portion (50a) of the first bag (50) at a position lower than second bag (54) to be hung.

2. The method for transferring the additive solution according to claim 1,
wherein the transfer line (55) includes a first bag-side tube (56) formed of a flexible material connected to the first bag (50), a second bag-side tube (60) formed of a flexible material connected to the second bag (54), and a branch section (62) formed to be harder than the first bag-side tube (56) and the second bag-side tube (60) and provided between the first bag-side tube (56) and the second bag-side tube (60), and
the support step arranges a liquid level (Lv1) inside the first bag (50) to be at a position lower than the branch section (62).

3. A method for transferring the additive solution targeted at a blood bag system (10) including a first bag (50) containing a blood component, a second bag (54) containing additive solution (M), and a transfer line (55) that forms a flow path between the first bag (50) and the second bag (54), the method being configured to transfer the additive solution (M) from the second bag (54) to the first bag (50),
the method for transferring the additive solution comprising:
a support step of supporting the first bag (50) or the transfer line (55) in a predetermined state such that, when the additive solution (M) is transferred from the second bag (54) to the first bag (50) using a height difference between the first bag (50) and the second bag (54), air from the second bag (54) stops in the middle of the flow path; and
a hanging step of hanging the second bag (54) such that an influx portion (54a) of the second bag (54) faces downward,
**characterized in that** the support step supports the first bag (50) and the transfer line (55) so as to form, in the transfer line (55), a high-level site (PI) existing at a position higher than the liquid level (Lvl) inside the first bag (50), and low-level sites (P2 and P3) existing at positions lower than the high-level site (P1), on both sides of the flow path of the high-level site (PI).

4. The method for transferring the additive solution according to claim 3,
wherein the transfer line (55) includes a first bag-side tube (56) formed of a flexible material connected to the first bag (50), a second bag-side tube (60) formed of a flexible material connected to the second bag (54), and a branch section (62) formed to be harder than the first bag-side tube (56) and the second bag-side tube (60) and provided between the first bag-side tube (56) and the second bag-side tube (60), and
the support step arranges the high-level site (PI) to be at a position lower than the branch section (62).

5. The method for transferring the additive solution according to any one of claims 1 to 4,
wherein the blood component is a concentrated red blood cell and the additive solution (M) is a red blood cell preservation.

## Patentansprüche

1. Verfahren zum Übertragen einer Additivlösung, die auf ein Blutbeutelsystem (10) gerichtet ist, das einen ersten Beutel (50), der eine Blutkomponente enthält, einen zweiten Beutel (54), der eine Additivlösung (M) enthält, und eine Übertragungsleitung (55), die einen Strömungsweg zwischen dem ersten Beutel (50) und dem zweiten Beutel (54) bildet, umfasst, wobei das Verfahren so konfiguriert ist, dass es die Additivlösung (M) von dem zweiten Beutel (54) zu dem ersten Beutel (50) überträgt,
wobei das Verfahren zur Übertragung der Additivlösung umfasst:
einen Stützschritt zum Stützen des ersten Beutels (50) oder der Übertragungsleitung (55) in einem vorbestimmten Zustand derart, dass, wenn die Additivlösung (M) unter Verwendung eines Höhenunterschieds zwischen dem ersten Beutel (50) und dem zweiten Beutel (54) von dem zweiten Beutel (54) zu dem ersten Beutel (50) übertragen wird, Luft aus dem zweiten Beutel (54) in der Mitte des Strömungsweges anhält; und
einen Aufhängeschritt zum Aufhängen des zweiten Beutels (54) derart, dass ein Einströmabschnitt (54a) des zweiten Beutels (54) nach unten weist,
**dadurch gekennzeichnet, dass** der Stützschritt den ersten Beutel (50) diagonal in Bezug auf eine horizontale Richtung stützt, um den ersten Beutel (50) so anzuordnen, dass er allmählich in Richtung eines Einströmabschnitts (50a) des ersten Beutels (50) an einer Position abgesenkt wird, die niedriger ist als die des zweiten aufzuhängenden Beutels (54).

2. Verfahren zur Übertragung der Additivlösung nach Anspruch 1,
wobei die Übertragungsleitung (55) einen ersten beutelseitigen Schlauch (56), der aus einem flexiblen Material gebildet ist, welcher mit dem ersten Beutel (50) verbunden ist, einen zweiten beutelseitigen Schlauch (60), der aus einem flexiblen Material gebildet ist, welcher mit dem zweiten Beutel (54) verbunden ist, und einen Verzweigungsabschnitt (62) aufweist, der härter als der erste beutelseitige Schlauch (56) und der zweite beutelseitige Schlauch (60) ausgebildet ist und zwischen dem ersten beutelseitigen Schlauch (56) und dem zweiten beutelseitigen Schlauch (60) vorgesehen ist, und
der Stützschritt einen Flüssigkeitspegel (Lv1) im Inneren des ersten Beutels (50) so anordnet, dass er sich an einer Position befindet, die niedriger als der Verzweigungsabschnitt (62) ist.

3. Verfahren zum Übertragen der Additivlösung, die auf ein Blutbeutelsystem (10) gerichtet ist, das einen ersten Beutel (50), der eine Blutkomponente enthält, einen zweiten Beutel (54), der eine Additivlösung (M) enthält, und eine Übertragungsleitung (55), die einen Strömungsweg zwischen dem ersten Beutel (50) und dem zweiten Beutel (54) bildet, umfasst, wobei das Verfahren so konfiguriert ist, dass es die Additivlösung (M) von dem zweiten Beutel (54) zu dem ersten Beutel (50) überträgt,
wobei das Verfahren zur Übertragung der Additivlösung umfasst:
einen Stützschritt zum Stützen des ersten Beutels (50) oder der Übertragungsleitung (55) in einem vorbestimmten Zustand derart, dass, wenn die Additivlösung (M) unter Verwendung eines Höhenunterschieds zwischen dem ersten Beutel (50) und dem zweiten Beutel (54) von dem zweiten Beutel (54) zu dem ersten Beutel (50) übertragen wird, Luft aus dem zweiten Beutel (54) in der Mitte des Strömungsweges anhält; und
einen Aufhängeschritt zum Aufhängen des zweiten Beutels (54) derart, dass ein Einströmabschnitt (54a) des zweiten Beutels (54) nach unten weist,
**dadurch gekennzeichnet, dass** der Stützschritt den ersten Beutel (50) und die Übertragungsleitung (55) so stützt, dass in der Übertragungsleitung (55) eine Stelle (P1) mit einem hohen Pegel, die an einer Position höher als der Flüssigkeitspegel (Lv1) im Inneren des ersten Beutels (50) vorhanden ist, und Stellen (P2 und P3) mit niedrigem Pegel, die an Positionen niedriger als die Stelle (P1) mit hohem Pegel vorhanden sind, auf beiden Seiten des Strömungsweges von der Stelle (P1) mit hohem Pegel gebildet werden.

4. Verfahren zur Übertragung der Additivlösung nach Anspruch 3,
wobei die Übertragungsleitung (55) einen ersten beutelseitigen Schlauch (56), der aus einem flexiblen Material gebildet ist, welcher mit dem ersten Beutel (50) verbunden ist, einen zweiten beutelseitigen Schlauch (60), der aus einem flexiblen Material gebildet ist, welcher mit dem zweiten Beutel (54) verbunden ist, und einen Verzweigungsabschnitt (62) aufweist, der härter als der erste beutelseitige Schlauch (56) und der zweite beutelseitige Schlauch (60) ausgebildet ist und zwischen dem ersten beutelseitigen Schlauch (56) und dem zweiten beutelseitigen Schlauch (60) vorgesehen ist, und
der Stützschritt die Stelle (P1) mit hohem Pegel so anordnet, dass sie sich an einer Position befindet, die niedriger als der Verzweigungsabschnitt (62) ist.

5. Verfahren zur Übertragung der Additivlösung nach einem der Ansprüche 1 bis 4,
wobei der Blutbestandteil ein konzentriertes rotes Blutkörperchen ist und die Additivlösung (M) eine Konservierungslösung für rote Blutkörperchen ist.

## Revendications

1. Procédé de transfert d'une solution d'additif destinée à un système de poches de sang (10) comportant une première poche (50) contenant un composant sanguin, une deuxième poche (54) contenant une solution d'additif (M) et une conduite de transfert (55) qui forme un chemin d'écoulement entre la première poche (50) et la deuxième poche (54), le procédé étant configuré pour transférer la solution d'additif (M) de la deuxième poche (54) à la première poche (50),
le procédé de transfert de la solution d'additif comprenant :
une étape de support consistant à supporter la première poche (50) ou la conduite de transfert (55) dans un état prédéterminé de sorte que, lorsque la solution d'additif (M) est transférée de la deuxième poche (54) à la première poche (50) en utilisant une différence de hauteur entre la première poche (50) et la deuxième poche (54), de l'air provenant de la deuxième poche (54) s'arrête au milieu du chemin d'écoulement ; et
une étape d'accrochage consistant à accrocher la deuxième poche (54) de sorte qu'une partie de flux entrant (54a) de la deuxième poche (54) soit orientée vers le bas,
**caractérisé en ce que** l'étape de support supporte la première poche (50) en diagonale par rapport à une direction horizontale pour agencer la première poche (50) pour qu'elle soit progressivement abaissée vers une partie de flux entrant (50a) de la première poche (50) à une position plus basse que la deuxième poche (54) à accrocher.

2. Procédé de transfert de la solution d'additif selon la revendication 1,
dans lequel la conduite de transfert (55) comporte un tube côté première poche (56) formé d'un matériau flexible relié à la première poche (50), un tube côté deuxième poche (60) formé d'un matériau flexible relié à la deuxième poche (54), et une section de bifurcation (62) formée pour être plus dure que le tube côté première poche (56) et le tube côté deuxième poche (60) et disposée entre le tube côté première poche (56) et le tube côté deuxième poche (60), et
l'étape de support agence un niveau de liquide (Lv1) à l'intérieur de la première poche (50) pour qu'il soit à une position plus basse que la section de bifurcation (62).

3. Procédé de transfert de la solution d'additif destinée à un système de poches de sang (10) comportant une première poche (50) contenant un composant sanguin, une deuxième poche (54) contenant une solution d'additif (M) et une conduite de transfert (55) qui forme un chemin d'écoulement entre la première poche (50) et la deuxième poche (54), le procédé étant configuré pour transférer la solution d'additif (M) de la deuxième poche (54) à la première poche (50),
le procédé de transfert de la solution d'additif comprenant :
une étape de support consistant à supporter la première poche (50) ou la conduite de transfert (55) dans un état prédéterminé de sorte que, lorsque la solution d'additif (M) est transférée de la deuxième poche (54) à la première poche (50) en utilisant une différence de hauteur entre la première poche (50) et la deuxième poche (54), de l'air provenant de la deuxième poche (54) s'arrête au milieu du chemin d'écoulement ; et
une étape d'accrochage consistant à accrocher la deuxième poche (54) de sorte qu'une partie de flux entrant (54a) de la deuxième poche (54) soit orientée vers le bas,
**caractérisé en ce que** l'étape de support supporte la première poche (50) et la conduite de transfert (55) de manière à former, dans la conduite de transfert (55), un site de niveau élevé (P1) existant à une position plus élevée que le niveau de liquide (Lv1) à l'intérieur de la première poche (50), et des sites de niveau bas (P2 et P3) existant à des positions plus basses que le site de niveau élevé (P1), sur les deux côtés du chemin d'écoulement du site de niveau élevé (P1).

4. Procédé de transfert de la solution d'additif selon la revendication 3,
dans lequel la conduite de transfert (55) comporte un tube côté première poche (56) formé d'un matériau flexible relié à la première poche (50), un tube côté deuxième poche (60) formé d'un matériau flexible relié à la deuxième poche (54), et une section de bifurcation (62) formée pour être plus dure que le tube côté première poche (56) et le tube côté deuxième poche (60) et disposée entre le tube côté première poche (56) et le tube côté deuxième poche (60), et
l'étape de support agence le site de niveau élevé (P1) pour qu'il soit à une position plus basse que la section de bifurcation (62).

5. Procédé de transfert de la solution d'additif selon l'une quelconque des revendications 1 à 4,
dans lequel le composant sanguin est un globule rouge concentré et la solution d'additif (M) est un milieu de conservation de globules rouges.
